# EUROPÄISCHE PATENTSCHRIFT

(11) **EP 0 346 741 B1**
(45) Veröffentlichungstag und Bekanntmachung des Hinweises auf die Patenterteilung: **10.02.1993**
(21) Anmeldenummer: 89110250.1
(22) Anmeldetag: 07.06.1989
(51) Int. Cl.: C07K 15/06, C07K 3/08, A61K 37/02, C12Q 1/56, C12N 9/72

(54) **Fibrin(ogen) derivate, Verfahren zu ihrer Herstellung und ihre Vewendung**
Fibrin(ogen) derivatives, process for preparing them and their use
Dérivés de fibrine ou de fibrinogène, leur procédé de préparation et leur application

(30) Priorität: 11.06.1988 DE 3819923
(43) Veröffentlichungstag der Anmeldung: 20.12.1989
(73) Patentinhaber: BEHRINGWERKE Aktiengesellschaft, 35001 Marburg (DE)
(72) Erfinder: Stief, Thomas, Dr., E-41007 Sevilla (ES)

(56) Entgegenhaltungen:
- BIOCHEMISTRY, Band 24, Nr. 14, Juli 1985, Seiten 3429-3434; B. CHIBBER et al.: "Effects of human fibrinogen and its cleavage products on activation of human plasminogen by streptokinase"

## Beschreibung

Die Erfindung betrifft ein Verfahren zur Herstellung von Derivaten von Fibrin, Fibrin- oder Fibrinogen-Spaltprodukten oder -Teilsequenzen, worin diese mit einem Oxidationsmittel behandelt werden, nach diesem Verfahren hergestellte Produkte und ihre Verwendung als Arzneimittel oder zur Diagnose.

Menschliches Blut verfügt über ein enzymatisches System, welches einmal entstandene Blutgerinnsel wieder aufzulösen vermag: das fibrinolytische System.

Das Zentralenzym der Fibrinolyse, das Plasmin, entsteht aus seiner Vorstufe Plasminogen über Aktivatoren, die aus Endothelzellen und anderen Zellverbänden freigesetzt werden. Die Aktivatoren können in zwei Typen eingeteilt werden: zum einen die Plasminogen-Aktivatoren vom Gewebetyp (genannt t-PA) zum anderen die Plasminogen-Aktivatoren vom Urokinasetyp (u-PA)

T-PA besitzt eine hohe Affinität zu Fibrin, Heparin und Plasmin- oder BrCN-Spaltprodukten des Fibrinogens und wird in deren Gegenwart in seiner plasminogenolytischen Aktivität stimuliert.

Solche t-PA-Stimulatoren sind in therapeutischer wie in diagnostischer Hinsicht von großem Interesse. Sie ermöglichen erhebliche Dosisreduktionen an zu verabreichendem t-PA und gestatten einen sensitiven t-PA-Nachweis in Plasma. Außerdem ermöglichen sie einfache affinitätschromatographische Reinigungen von t-PA aus biologischen Flüssigkeiten wie z.B. Zellüberstand.

Stimulatoren wie BrCN-Spaltprodukte des Fibrinogens sind allerdings aufgrund ihrer hohen Toxizität für den therapeutischen Einsatz nicht zu verwenden. Heparin stellt sich in vivo als wenig effizienter Stimulator für t-PA dar.

Durch Sekretion von Proteinasen, u.a. Urokinase, Collagenase und Elastase, sowie von Oxidationsprodukten, hier im besonderen N-Chloramine, beteiligen sich polymorphkernige Leukozyten und Makrophagen an der physiologischen Fibrinolyse.

Überraschenderweise wurde nun gefunden, daß die durch N-Chloramine, wie das Chloramin T, bedingte Oxidation von Fibrin, Fibrin- oder Fibrinogen-Spaltprodukten (FSP) zu einer drastischen (ca. 5- bis 10fachen) Verbesserung der t-PA-stimulierenden Eigenschaften dieser Produkte führt (Stimulation unoxidierte FSP = ca. 5fach, Stimulation oxidierte FSP = ca. 25fach, verglichen mit t-PA ohne Stimulator). Es zeigte sich, daß der D-Bereich des Fibrinogens die für diesen Effekt verantwortliche Domäne darstellt.

Gegenstand der Erfindung sind daher Derivate von Fibrin, Fibrin- oder Fibrinogen-Spaltprodukten oder -Teilsequenzen, dadurch gekennzeichnet, daß in den zugrundeliegenden Aminosäure-Sequenzen die Methionin-Reste alle oder zum Teil in Methioninsulfoxid-Reste umgewandelt sind.

Gegenstand der Erfindung ist auch ein Verfahren zur Herstellung von Derivaten von Fibrin, Fibrin- oder Fibrinogen-Spaltprodukten oder -Teilsequenzen gekennzeichnet dadurch, daß Fibrin, Fibrin- oder Fibrinogen-Spaltprodukte oder -Teilsequenzen mit einem Oxidationsmittel behandelt werden.

Gegenstand der Erfindung ist auch ein Verfahren, worin Fibrinogen oder Fibrin mit einem Oxidationsmittel behandelt und das Produkt gespalten wird. Vorzugsweise wird enzymatisch gespalten, und zwar z.B. durch Einwirkung von Plasmin, Thrombin oder Batroxobin auf oxidiertes Fibrinogen.

Lösliches Fibrin (sog. Fibrinmonomere) als Ausgangsmaterial wird z.B. hergestellt, indem 1 mg/ml Faktor XIII-freies Fibrinogen, z.B. F XIII-Reagenz, Behringwerke, in 25 mmol/l Tris, 5 mmol/l CaCl₂, 2 mmol/l Gly-Pro-Arg-Pro, pH 7,8, mit 0,5 IU Test-Thrombin 10 min bei 37°C inkubiert wird und 5 IU Hirudin/ml zugesetzt werden.

Fibrinogen-Spaltprodukte können gemäß dem von Stief et al. (Thromb.Res. 48, 603-609, 1987) beschriebenen Verfahren erzeugt werden. Hierzu wird eine Fibrinogenlösung (1-10 mg/ml) in 25 mmol/l Tris, 5 mmol/l EDTA, pH 7,8, durch Plasmineinwirkung abgebaut, vorzugsweise durch Passieren der Fibrinogenlösung durch eine Plasminsepharose (2,5 mg Protein/ml Gel) bei einer Laufgeschwindigkeit von ca. ml/h statt.

Die Oxidation der Ausgangsmaterialien (= Fibrinogenlösung oder die entstandenen Fibrinmonomere bzw. Spaltprodukte) wird durchgeführt bei pH 7-9,5, vorzugsweise in einem Puffer, besonders Trispuffer, gegebenenfalls unter Zusatz von 0,01-0,2, vorzugsweise 0,1 % (w/v) Detergenz wie ^{R}Triton X 100 und gegebenenfalls zur Löslichkeitsverbesserung unter Zusatz von Mannit, vorzugsweise 5-20mmol/l. Als Oxidationsmittel wird vorzugsweise ein Chloramin, wie Chloramin T, verwendet, und zwar in einer Konzentration von 1-20 mmol/l, vorzugsweise 5-10 mmol/l. Die Oxidation kann durch Zusatz eines Überschusses an Reduktionsmittel, wie Ascorbinsäure, Acetylcystein oder Acetylmethionin beendet werden. Die Oxidation kann durchgeführt werden bei 4-45°C, vorzugsweise 20-40°C, und verläuft innerhalb von Sekunden (weniger als 5 min).

Die Endprodukte sind dadurch gekennzeichnet, daß bei einer durchzuführenden Aminosäureanalyse Methionin nach Proteinoxidation nur noch in Spuren, dafür jedoch Methioninsulfoxid in Mengen nachgewiesen werden, die dem im Protein vorkommenden Gehalt an Methionin entspricht. Die plasmingespaltenen und oxidierten Derivate besitzen außerdem eine bessere Löslichkeit in aqua dest. als die unoxidierten.

Auch synthetische methioninhaltige Peptide mit Sequenzen aus dem D-Bereich des Fibrinogens sind als Ausgangsmaterial für das erfindungsgemäße Verfahren geeignet. Oxidierte FSP binden t-PA und sc-uPA (Einkettenform-Urokinase) affiner als nichtoxidierte, wie in Versuchen mit an ^{R}Sepharose 48 kovalent gebundenen FSP gezeigt werden konnte. Von daher eignen sich oxidierte Fibrin-bzw. Fibrinogenderivate in besonderer Weise, um t-PA oder sc-uPA z.B. aus Zellüberständen affinitätschromatographisch zu reinigen.

Als Oxidationsmittel sind besonders methioninspezifische Oxidantien, die vorzugsweise im alkalischen Methionin in Methioninsulfoxid umwandeln, geeignet, vorzugsweise Chloramine (z.B.Chloramin T oder Chloramin B), oder Salze der hypochlorigen Säure (z.B. NaOCl). Zur Umwandlung von Methionin in Methioninsulfoxid eignen sich nach Savige und Fontana (Methods in Enzymology 47: 453-459, 1977) Farbstoff (z.B. Methylenblau)-vermittelte Photooxidation sowie Bromsuccinimid; N-Chlorsuccinimid; 2,4,5-Tribromo-4-methylcyclohexadienon; BNPS-Skatol (2-(2-nitrophenylsulfonyl)-3-methyl-3-bromoindolamin); Chloramin T; t-Butylhypochlorit; Trichlormethansulfonyl-chlorid, ferner 1-Chlorobenzotriazol, Jodobenzenedichlorid in wässrigem Pyridin,Pyridinbromidkomplexe, Quinoline und besonders 1,4-Diazobicyclo (2.2.2) octane in wässriger Essigsäure.

Die Oxidation, vorzugsweise bei pH 8-9, wird durch Zusatz von 0,05-0,1 % Detergenz wie ^{R}Triton X 100 verbessert.

Oxidierte FSP binden sich ebenfalls an Fibrin wesentlich affiner als nichtoxidierte, wie in Versuchen mit zur Gerinnung gebrachtem Plasma gezeigt werden kann. Von daher eignen sie sich in besonderer Weise, um für Thrombusdiagnose und -therapie interessante Substanzen an ein Gerinnsel in vivo zu bringen.

Nach dem Stand der Technik werden Thrombi unter Verwendung von radioaktiv markiertem Fibrinogen in vivo nachgewiesen (Mahn, I., Müller-Berghaus, G. Haemostatis, 4: 40:50, 1975). Da oxidierte FSP wesentlich höhere Affinität zum Thrombus besitzen als Fibrinogen, ist die Verwendung von radioaktiv markierten oxidierten FSP, vorzugsweise FSP-D, vorteilhafter, da 1. innerhalb kürzerer Zeit (weniger als 1 h) nach Applikation des Diagnostikums der klinische Befund vorliegt und 2. der Gesamtorganismus mit niedrigeren radioaktiven Dosen belastet wird, da man aufgrund der hohen Affinität weniger markiertes Protein benötigt.
Zum anderen werden Plasminogen-Aktivatoren (PA) nach dem Stand der Technik an Fibringerinnsel transportiert, indem man kovalent an Fibrinantikörper gebundene PA oder gentechnische Chimäre aus PA und Fibrinantikörper verwendet.

Nachteile dieser Methoden liegen in der Induktion von Antikörpern gegen das körperfremde Protein. Durch die Verwendung von menschlichen oxidierten FSP lassen sich die damit verbundenen Nebenwirkungen (z.B. Immunkomplexkrankheiten) vermeiden.

Mittels oxidierten FSP lassen sich auch HOCl und Chloramin produzierende Enzyme wie Myeloperoxidase, Glucoseoxidase, Xanthinoxidase, falls sie sich in kovalent gebundenem Zustand mit oxidierten FSP befinden, an einen Thrombus transportieren. Die Enzymreaktion kann gestartet werden durch Infusion geeigneter untoxischer Substrate (z. B. Glucose, Xanthin, Hypoxanthin) Oxidation von Fibrinpolymeren in vivo erniedrigt den körpereigenen und evtl. fremden Bedarf an t-PA zur Auflösung eines Thrombus, wodurch, wenn überhaupt, für eine erfolgreiche Lyse lediglich geringe Mengen an t-PA verabreicht werden müssen. Die damit verbundene oxidative Inaktivierung von im Thrombus gebundenen Fibrinolyseinhibitor Alpha-2-Antiplasmin verstärkt die profibrinolytischen Effekte.

Darüber hinaus kommt es durch intravenöse Applikation von oxidierten FSP zu einer verstärkten generalisierten Stimulation von t-PA und sc-uPA, wodurch diese PA in der Dosis reduziert oder ganz auf ihren Einsatz verzichtet werden kann.

Im weiteren eignen sich oxidierte Fibrin(ogen)-Derivate, um stimulierbare Plasminogen-Aktivatoren wie t-PA in in-vitro-Testsystemen in Plasma oder anderen biologischen Flüssigkeiten sensitiv und spezifisch nachzuweisen. Hierzu werden beispielsweise 50-200 µg oxidierte Fibrinogenspaltprodukte/ml Reaktionsansatz in einem chromogenen Nachweisverfahren für t-PA zusammen mit 3 CTA-U Plasminogen und 0,3 µM eines chromogenen Plasminsubstrates eingesetzt.

### Beispiel 1

### Herstellung der oxidierten Fibrinogenspaltprodukte

### a) Plasminspaltprodukte

250 mg Testfibrinogen (Behringwerke) gelöst in 50 ml aqua dest. mit 5 mmol/l EDTA wurden über 25 ml Plasmin-^{R}Sepharose 4B (2,5 mg Plasmin/ml Gel) bei einer Fließgeschwindigkeit von 60 ml/h bei Raumtemperatur (RT) gegeben. Der Durchlauf (DL) wurde auf pH 8,5 eingestellt, 12,5 ml 100 mmol/l Chloramin T zugesetzt und 15 min bei 37°C inkubiert. Danach wurde der Oxidationsansatz gegen 200faches Volumen 50 mmol/l Tris, pH 8,5 dialysiert (48 h, 4°C).

Alternativ konnten die oxidierten Spaltprodukte durch eine vor der Plasmin-Sepharose-Passage stattfindende Oxidation und anschließende Plasminproteolyse erhalten werden.

### b) Thrombinspaltprodukte

Erfindungsgemäße Thrombinspaltprodukte oxidierten Fibrinogens werden erhalten, indem 1 mg/ml Fibrinogen zunächst mit Chloramin T bei einer Konzentration von 2,5 mmol/l oxidiert und anschließend mit 0,5 IU Test-Thrombin (Behringwerke) 10 min bei 37°C und pH 7,8 inkubiert wird und 5 IU Hirudin/ml zugesetzt werden.

### Beispiel 2

### Stimulation der plasminogenolytischen Aktivität von t-PA durch FSP in Abhängigkeit der Oxidationsmittelkonzentration - Nachweis von t-PA durch oxidierte FSP

a) 100 µg FSP-BrCN (über BrCN-Spaltung hergestellte FSP) bzw. 100 µg FSP-EDTA (über Plasminspaltung in Anwesenheit von EDTA hergestellte FSP) in 100 µl 50 mmol/l Tris, 100 mmol/l NaCl, 0,01 % ^{R}Triton X 100, pH 8,5 und Trispuffer ohne FSP wurden mit 50 µl Chloramin T unterschiedlicher Konzentration (0-20 mmol/l) in aqua dest. 10 min bei 37°C inkubiert. Nach Zugabe von 100 µl 3 mmol/l HD-Nva-CHA-Lys-pNA in aqua dest., 200 µl 1,3 µmol/l Glu-Plasminogen in 100 mmol/l Tris, 100 mmol/l NaCl, 1 % Haemaccel, 0,1 % ^{R}Triton X 100, pH 8,4 (TNHT-Puffer) und 10 IE Zweiketten-t-PA in 200 µl TNHT-Puffer erfolgte eine weitere Inkubation über 12 min bei 37°C. Die Substratumsetzung wurde durch Zusatz von 500 µl 3,4 mol/l Essigsäure beendet und die entstandene Absorptionsänderung bei 405 nm detektiert.
b) In einer Variation wurde die Oxidation der FSP bei Zusatz von 50 µl 10 mmol/l Chloramin T nach 10 min (37°C) Vorinkubation durch Zugabe von 50 µl 5 mmol/l Dithiotreitol beendet und anschließend die Plasminogenaktivierung durchgeführt.

Ergebnis aus Beispiel 2a,b:

**Tabelle 1**

| Chloramin T (Konz. in 50 µl-Zugabe) | Fibrinogenspaltprodukte | | |
|---|---|---|---|
| | BrCN-gespalten | mit EDTA über Plasmin-Seph. | ohne FSP |
| | Plasminaktivität (A_{405 nm}; mE) | | |
| 0 mmol/l | 898 | 1053 | 122 |
| 1 mmol/l | 1007 | 947 | 132 |
| 2,5 mmol/l | 1067 | 1310 | 134 |
| 5 mmol/l | 914 | 1936 | 135 |
| 7,5 mmol/l | 728 | 2218 | 136 |
| 10 mmol/l | 517 | 2315 | 132 |
| 15 mmol/l | 220 | 2091 | 118 |
| 20 mmol/l | 138 | 1900 | 98 |
| 10 mmol/l /5 mmol/l DTT | 806 | 2356 | 129 |

Steigende Konzentrationen an Chloramin T führen zu einer gesteigerten t-PA-Stimulierbarkeit. FSP-EDTA, FSP-BrCN zeigen nur eine minimale (10 %) und nur bei niedriger Chloramin T-Konzentration vorkommende Verbesserung ihrer Stimulation gegenüber t-PA. Höhere Chloramin T-Konzentrationen führen zu einer Verschlechterung der t-PA-Stimulation.

### Beispiel 3

### Stimulation der plasminogenolytischen Aktivität von t-PA durch unterschiedliche FSP - t-PA-Nachweis durch FSP

Je 100 µg FSP-BrCN, FSP-EDTA, Fibrinogen sowie FSP-EDTA, erhalten durch Passage von oxidiertem Fibrinogen (siehe Beispiel 1) durch eine Plasmin-^{R}Sepharose, wurden gemäß der in Beispiel 2a) aufgeführten Testbedingungen bei Zusatz von 50 µl 20 mmol/l Chloramin T und aqua dest. zur Kontrolle auf deren Stimulierungskraft gegenüber Zweiketten-t-PA untersucht. Die Oxidation wurde nach 10 min (37°C) durch Zugabe von 100 µl 6,25 mmol/l Dithiotreitol, die Substratumsetzung nach 9 min (37°C) durch Zusatz von 500 µl 3,4 mol/l Essigsäure beendet.

Ergebnis siehe Tabelle 2.

Oxidation im Test führt im Falle der FSP-EDTA zu einer ausgeprägten Steigerung der t-PA-Stimulationskraft. FSP-BrCN und FSP-EDTA aus voroxidiertem Fibrinogen besitzen bereits vor der Testoxidation mehr als 10fache Stimulationspotenz gegenüber der Pufferkontrolle. Auch die Oxidation von Fibrinogen erhöht dessen Stimulations-potential.

**Tabelle 2**

| Stimulans | aqua dest. | 50 µl 20 mmol/l Chloramin T |
|---|---|---|
| | Plasminaktivität (A_{405 nm}; mE) | |
| FSP-BrCN | 443 | 614 |
| FSP-EDTA | 276 | 1172 |
| FSP-EDTA (aus voroxidiertem Fibrinogen) | 485 | 962 |
| Fibrinogen | 117 | 156 |
| Pufferkontrolle | 30 | 27 |

### Beispiel 4

### Affinitätschromatographische Reinigung von t-PA und sc-uPA durch immobilisierte oxidierte Fibrin- oder Fibrinogen-Spaltprodukte und -Derivate

1 ml Aliquote von single chain (sc)-t-PA, sc-uPA, two chain (tc)-uPA (1 µg/ml) in TNHT-Puffer wurden 45 min bei Raumtemperatur mit jeweils 0-300 Ul einer 50 %igen FSP-EDTA-Lösung oder oxidierten FSP-EDTA-^{R}Sepharose 4B (2 mg Protein/ml Gel) unter leichtem Schütteln inkubiert. Nach Abzentrifugation wurden die Überstände entfernt und auf deren plasminogenolytische Aktivität getestet. Die verbliebene Aktivität wurde in Prozent der Ausgangsaktivität ausgedrückt. Zu der im Bodensatz verbliebenen ^{R}Sepharose 4B wurde die 100fach Menge TNHT-Puffer gegeben, durchgemischt und erneut zentrifugiert. Der überstand wurde dekantiert und 1 ml Elutionsmedium zugegeben. Hierfür wurden 2M KSCN und 1 % Natriumdodecylsulfat verwendet. Nach einer 10minütigen Inkubation (RT) unter Schütteln wurde abzentrifugiert und gegen das 200fache Volumen an TNHT-Puffer dialysiert (48 h, 4°C) Anschliessend wurden die Proben auf ihre plasminogenolytische Aktivität untersucht.

Plasminogenolytische Aktivtäten wurden bestimmt durch Inkubation von 50 µl Probe (Überstand) mit 100 µg FSP-BrCN, 278 pM Glu-Plasminogen, 0,3 µmol HD-Nva-CHA-Lys-pNA in einem Volumen von 600 µl für 5 min bei 37°C. Die Substratumsetzung wurde durch Zusatz von 500 µl 3,4 mol/l Essigsäure beendet und die entstandene Extinktion bei 405 nm bestimmt.

### Ergebnis:

Immobilisierte oxidierte FSP-EDTA zeigen eine wesentlich höhere Affinität zu t-PA als unoxidierte. Im Gegensatz zu u-PA bindet sc-uPA affin an oxidierte FSP. Die Elution der gebundenen PA-Moleküle kann durch 2 mol/l KSCN oder 1 % SDS erfolgen.

**Tabelle 3**

| zugesetzte oxidierte (nicht oxidierte) 50%ige FSP-Sepharose (µl) | Restaktivität im Überstand | | |
|---|---|---|---|
| | sc-t-PA | sc-u-PA | tc-u-PA |
| | (%) der Ausgangsaktivität | | |
| 0 | 100 (100) | 100 (100) | 100 (100) |
| 20 | 97 (100) | 100 (100) | 100 (98) |
| 50 | 85 (100) | 94 (100) | 100 (100) |
| 100 | 55 (87) | 91 (97) | 97 (100) |
| 200 | 47 (81) | 78 (92) | 96 (99) |
| 300 | 31 (47) | 55 (90) | 90 (96) |

### Beispiel 5

### Trennen der oxidierten und nicht-oxidierten Fibrinogenspaltprodukte über DEAE-Sephacel und anschließende Polyacrylamidgel-Elektrophorese (PAGE) und Stimulationstestung der einzelnen Fragmente

Der Durchlauf der Plasminsepharose, wie in Beispiel 1 angegeben, wurde gegen das 200fache Volumen 10 mmol/l Natriumphosphatpuffer, pH 8,6 umdialysiert. Anschließend wurde das Dialysat auf 100 ml der mit dem 3fachen Säulenvolumen 10 mmol/l Natriumphosphat vorequilibrierten DEAE-Sephacel aufgetragen. Bei einer Laufgeschwindigkeit von 60 ml/h bei Raumtemperatur wurde mittels eines steigenden Ionenstärke- und fallenden pH-Gradienten eluiert (10 mmol/l Natriumphosphat, pH 8,6, 300 mmol/l Kaliumhydrogenphosphat, pH 4,3, jeweils 100 ml). 3 ml Fraktionen wurden gesammelt.

Durchlauf (vor Anschluß des Gradienten) und einzelne Peaks wurden bezüglich optischer Dichte bei 280 nm sowie in Natriumdodecylsulfat-PAGE untersucht (Ergebnis siehe Tabellen 4 und 5). Fibrinogenspaltprodukte durch Plasminspaltung besitzen folgendes ungefähres Molekulargewicht (KDa) : Fibrinogen 340, X 300, Y 150, D 90, 87, 85, E 60.

**Tabelle 4**

| Nichtoxidiertes Ausgangsmaterial | | | |
|---|---|---|---|
| Fraktion-Nr. | | OD₂₈₀ₙₘ | Hauptbande (Nebenbande) im SDS-Gel, unreduziert (KDa) |
| Durchlauf | 14 - 29 | 0,7 | 90 |
| Peak 1 | 47 - 50 | 4,4 | 90, 85 |
| Peak 2 | 51 - 54 | 5,6 | 90 (300, 60, 150) |
| Peak 3 | 55 - 58 | 0,46 | 90, 60 (300, 150) |
| Peak 5 | 83 - 89 | 0,3 | 60, 300 (150) |
| Peak 6 | 90 - 98 | 0,32 | 60 (300) |
| Peak 7 | 109 -113 | 0,1 | (90, 300) |
| Peak 1 entspricht Fibrinogen-Fragment D. | | | |

**Tabelle 5**

| Oxidiertes Ausgangsmaterial | | | |
|---|---|---|---|
| Fraktion-Nr. | | OD₂₈₀ₙₘ | Hauptbande (Nebenbande) im SDS-Gel, unreduziert (KDa) |
| Durchlauf | 12 - 30 | 0,34 | (90, 60, 150, 300) |
| Peak 1 | 53 - 55 | 0,4 | 90 |
| Peak 2 | 56 - 60 | 0,98 | 85 |
| Peak 3 | 61 - 67 | 2,2 | 90 |
| Peak 4 | 85 - 98 | 0,72 | 60, 300, 150 |
| Peak 5 | 109 -120 | 0,08 | 300 (90) |
| Peak 1 - 3 entsprechen Fibrinogen-Fragment D. | | | |

100 µl der einzelnen Fraktionen der DEAE-Trennung unoxidierter FSP (EDTA) wurden gemäß Beispiel 2 auf deren t-PA-stimulierende Aktivität im unoxidierten und oxidierten Zustand untersucht. Ergebnis siehe Tabelle 6.

**Tabelle 6**

| Zusatz von 50 µl | a) aqua dest. | b) 20 mmol/l Chloramin T |
|---|---|---|
| | Plasminaktivität A_{405 nm}/9 min(mE) | |
| Durchlauf (Hauptbande in SDS-Gel) | 184 | 703 |
| Peak 1 (FSP-D) | 320 | 1890 |
| Peak 2 (FSP-D) | 451 | 1728 |
| Peak 3 (FSP-D, E) | 583 | 1145 |
| Peak 5 (E, X) | 266 | 223 |
| Peak 6 (E) | 197 | 291 |

An den gemessenen Plasminaktivitäten wird erkannt, daß die für die Stimulation wie für die Oxidation verstärkte Stimulation verantwortliche Domäne des Fibrinogen-Moleküls der D-Bereich ist.

### Beispiel 6

### Aminosäurenanalyse von oxidiertem und unoxidiertem FSP-D

Fibrinogenspaltprodukt D (Peak 1, gereinigt gemäß Beispiel 5) 1 mg/ml wurde gemäß Beispiel 1 in 50 mmol/l Tris-Puffer, pH 8,5 mit und ohne Zusatz von 5 mmol/l Chloramin T 30 min bei 37°C inkubiert. Darauhin wurde gegen das 100fache Volumen aqua dest. 48 h bei 4°C dialysiert und in einem LKB 4150 Aminosäurenanalysator unter variierten Standard-Hydrolyse-Bedingungen (24 h, 110°C in vacuo, 3N p-toluene sulfonic acid) 1,2 nM Protein pro Analyse eingesetzt. Die Ergebnisse sind Durchschnittswerte von Dreifachbestimmungen. Unter den benutzten Bedingungen konnte die Aminosäure Tryptophan nicht bestimmt werden (Tabelle 7).

**Tabelle 7**

| Aminosäurenzusammensetzung | D (%) | oxid. D (%) |
|---|---|---|
| Asp | 13.2 | 12.5 |
| Thr | 4.0 | 4.3 |
| Ser | 5.8 | 6.1 |
| Glu | 10.3 | 12.4 |
| Pro | 4.9 | 5.6 |
| Gly | 11.6 | 12.2 |
| Ala | 4.7 | 4.5 |
| Cys | 0.4 | 0.4 |
| Val | 3.9 | 4.4 |
| Met | 0.8 | <0.1 |
| Ile | 3.3 | 3.5 |
| Leu | 5.3 | 5.6 |
| Tyr | 2.8 | 2.4 |
| Phe | 3.1 | 3.0 |
| His | 3.2 | 3.3 |
| Lys | 8.4 | 8.2 |
| Arg | 6.3 | 6.9 |

Beide Proteine unterscheiden sich im wesentlichen durch das Fehlen von Methionin im oxidierten D. Methioninsulfoxid wandert im Analysator an anderer Stelle und wird nicht als Methionin angezeigt.

### Beispiel 7

### Erhöhte Affinität zu Fibrinthromben von oxidierten FSP gegenüber unoxidierten: Nachweis von Thromben

0,5 ml Plasma wurde durch Zugabe von 50 µl 0,2 mol/l CaCl₂ und 0,5 IE Thrombin zur Gerinnung gebracht. Anschließend wurden die erhaltenen Gerinnsel durch dreimaliges Zentrifugieren in der 20fachen Menge Phosphate Buffered Saline (PBS) gewaschen und in je einer PBS-Lösung von 0,5 ml oxidierten FSP (FSP = Plasminabbau von Fibrinogen in Anwesenheit von 5 mmol/l EDTA) (1 mg/ml), 0,5 ml unoxidierten FSP (1 mg/ml) bzw. 0,5 ml Fibrinogen (1 mg/ml) für 1 Stunde bei Raumtemperatur inkubiert (Kontrolle = FSP-Lösungen ohne Gerinnsel). Danach wurde die im Überstand verbliebene Stimulationskraft der FSP getestet, indem 50 µl einer Gewebe-Plasminogen-Aktivatorlösung (500 IE tc-t-PA/ml) in PBS mit 100 µl Überstand, 200 µl Plasminogen (= 1 CTA-U in 100 mmol/l Tris, 100 mmol/l NaCl, 1 % (w/v) ^{R}Haemaccel, 0,1 % (w/v) ^{R}Triton X 100, pH 8.4) und 100 µl 3 mmol/l D-Norvalyl-cyclohexyl-alanyl-lysyl-paranitroanilide (HD-Nva-CHA-Lys-pNA) in aqua dest. 8 min bei 37°C inkubiert wurden, mit 500 µl 8,5 mol/l Essigsäure abgestoppt und die Extinktion bei 405 nm bestimmt wurde.

Ergebnis siehe Tabelle 8.

**Tabelle 8**

| Probe = Stimulus (1-3) | Aktivität Plasmin (t-PA) A_{405 nm}/8 min (mE) | t-PA-Stimulationskraft* |
|---|---|---|
| ohne Gerinnsel: | | |
| 1 oxid. FSP | 1 235 ± 14 | 15,4 |
| 2 unoxid. FSP | 587 ± 6 | 7,3 |
| 3 Fibrinogen | 216 ± 1 | 2,7 |

| mit Gerinnsel: | | |
|---|---|---|
| 1 oxid. FSP | 755 ± 4 | 61 %** |
| 2 unoxid. FSP | 465 ± 1 | 80 % |
| 3 Fibrinogen | 171 ± 2 | 80 % |
| Kontrolle (Stimulus) prozentual | 80 ± 3 | |

| | | |
|---|---|---|
| * Verhältnis t-PA Aktivität mit Stimulus: t-PA Aktivität ohne Stimulus | | |
| ** in % ohne Gerinnsel | | |

Man erkennt, daß der größte Abfall der t-PA-Stimulationskraft des Überstandes bei oxid. FSP eintritt, was bedeutet, daß oxid. FSP affiner an Fibrin binden als unoxid. FSP oder Fibrinogen.

### Beispiel 8

### Oxidation von Fibrinmonomeren führt zu einer Verstärkung ihrer Stimulationseigenschaften gegenüber t-PA

100 µl (1 mg/ml) Fibrinmonomere in Puffer wurden mit Chloramin T unterschiedlicher Konzentration 10 min bei 37°C inkubiert.Die Oxidation wurde daraufhin durch Zugabe von 100 µl mmol/l N-Acetyl-Methionin, pH 8.4, beendet, 200 µl tc-t-PA (= 10 IE) in Grundpuffer U, 200 µl Plasminogen (1 CTA-U) in Grundpuffer U und 100 µl 3 mmol/l HD-Nva-CHA-Lys-pNA in aqua dest. zugefügt und 24 min bei 37°C inkubiert.

Die Substratumsetzung wurde durch Zusatz von 500 µl 8,5 mol/l Essigsäure beendet und die entstandene Extinktion bei 405 nm bestimmt.

Ergebnis:

**Tabelle 9**

| Chloramin T-Zusatz (50 µl) (mmol/l) | Aktivität Plasmin (mA) | |
|---|---|---|
| | Fibrinmonomere | Puffer |
| 0 | 1274 ± 5 | 224 ± 3 |
| 1 | 551 ± 5 | |
| 5 | 795 ± 11 | |
| 10 | 2095 ± 45 | |
| 20 | 2287 ± 4 | |
| 40 | 2276 ± 4 | 248 ± 8 |

Man erkennt, daß steigende Konzentrationen an Chloramin nach anfänglicher Erniedrigung später zu einer Erhöhung der t-PA-Stimulation führen. Zusatz von Methionin beeinträchtigt das Meßverfahren nicht, wie in der Pufferkontrolle gezeigt werden kann.

## Patentansprüche (Patentansprüche für folgende(n) Vertragsstaat(en): AT, BE, CH, DE, FR, GB, IT, LI, LU, NI, SE)

1. Derivat von Fibrin, Fibrin- oder Fibrinogen-Spaltprodukten oder -Teilsequenzen, dadurch gekennzeichnet, daß in den zugrundeliegenden Aminosäure-Sequenzen die Methionin-Reste alle oder zum Teil in Methioninsulfoxid-Reste umgewandelt sind.

2. Derivat nach Anspruch 1, dadurch gekennzeichnet, daß die Spaltprodukte oder Teilsequenzen die D-Domäne oder Teilsequenzen daraus enthalten.

3. Derivat nach Anspruch 1 als Arzneimittel.

4. Fibrinogen, in dem die Methionin-Feste alle oder zum Teil in Methioninsulfoxid umgewandelt sind, als Arzneimittel.

5. Verfahren zur Herstellung eines Derivats nach Anspruch 1, dadurch gekennzeichnet, daß Fibrin, ein Fibrin-oder Fibrinogen-Spaltprodukt oder eine Fibrin- oder Fibrinogen-Teilsequenz mit einem Oxidationsmittel unter Bedingungen behandelt wird, daß die in der Aminosäure-Sequenz dieser Stoffe enthaltenen Methionin-Reste alle oder zum Teil in Methioninsulfoxid-Reste umgewandelt werden.

6. Verfahren zur Herstellung eines Derivats nach Anspruch 5, wobei es sich bei dem Oxidationsmittel um ein Halogenamin, vorzugsweise Chloramin, handelt.

7. Verfahren nach Anspruch 5, dadurch gekennzeichnet, daß Spaltprodukte verwendet werden, die in Anwesenheit von Chelatbildnern hergestellt wurden.

8. Verfahren nach Anspruch 5, dadurch gekennzeichnet, daß dem Reaktionsgemisch ein Detergenz zugesetzt wird.

9. Verfahren nach Anspruch 5, dadurch gekennzeichnet, daß die Oxidation bei pH 7 - 9.5 durchgeführt wird.

10. Verwendung eines Derivats nach Anspruch 1 zur Diagnose.

11. Verwendung eines Derivats nach Anspruch 1 als affines Agenz.

12. Verwendung von Fibrinogen, in dem die Methionin-Reste alle oder zum Teil in Methioninsulfoxid-Reste umgewandelt sind als affines Agenz.

## Patentansprüche (Patentansprüche für folgende(n) Vertragsstaat(en): ES)

1. Verfahren zur Herstellung eines Derivats von Fibrin, Fibrin- oder Fibrinogen-Spaltprodukten oder -Teilsequenzen, dadurch gekennzeichnet, daß Fibrin, ein Fibrin- oder Fibrinogen-Spaltprodukt oder eine Fibrin- oder Fibrinogen-Teilsequenz mit einem Oxidationsmittel unter Bedingungen behandelt wird, daß die in der Aminosäure-Sequenz dieser Stoffe enthaltenen Methionin-Reste alle oder zum Teil in Methioninsulfoxid-Reste umgewandelt werden.

2. Verfahren nach Anspruch 1, wobei es sich bei dem Oxidationsmittel um ein Halogenamin, vorzugsweise Chloramin, handelt.

3. Verfahren nach Anspruch 1, dadurch gekennzeichnet, daß Spaltprodukte verwendet werden, die in Anwesenheit von Chelatbildnern hergestellt wurden.

4. Verfahren nach Anspruch 1, dadurch gekennzeichnet, daß die Spaltprodukte oder Teilsequenzen die D-Domäne oder Teilsequenzen daraus enthalten.

5. Verfahren nach Anspruch 1, dadurch gekennzeichnet, daß dem Reaktionsgemisch ein Detergenz zugesetzt wird.

6. Verfahren nach Anspruch 1, dadurch gekennzeichnet, daß die Oxidation bei pH 7 - 9.5 durchgeführt wird.

## Claims (Claims for the following Contracting State(s): AT, BE, CH, DE, FR, GB, IT, LI, LU, NL, SE)

1. A derivative of fibrin, or fibrin or fibrinogen degradation products or partial sequences, wherein all or some of the methionine residues in the basic amino acid sequences have been converted to methionine sulfoxide residues.

2. A derivative as claimed in claim 1, wherein the degradation products or partial sequences contain the D domain or partial sequences thereof.

3. A derivative as claimed in claim 1 as a medicament.

4. Fibrinogen in which all or some of the methinone (sic) residues have been converted to methionine sulfoxide, as a medicament.

5. A process for the preparation of a derivative as claimed in claim 1, which comprises treating fibrin, a fibrin or fibrinogen degradation product or a fibrin or fibrinogen partial sequence with an oxidizing agent under conditions such that all or some of the methionine residues contained in the amino acid sequence of these substances are converted to methionine sulfoxide residues.

6. The process for the preparation of a derivative as claimed in claim 5, wherein the oxidizing agent is a halogenoamine, preferably chloramine.

7. The process as claimed in claim 5, wherein degradation products are used which have been prepared in the presence of chelate-forming agents.

8. The process as claimed in claim 5, wherein a detergent is added to the reaction mixture.

9. The process as claimed in claim 5, wherein the oxidation is carried out at pH 7 - 9.5.

10. The use of a derivative as claimed in claim 1 for diagnosis.

11. The use of a derivative as claimed in claim 1 as an affinity agent.

12. The use of fibrinogen in which all or some of the methionine residues have been converted to methionine sulfoxide residues as an affinity agent.

## Claims (Claims for the following Contracting State(s): ES)

1. A process for the preparation of a derivative of fibrin, or fibrin or fibrinogen degradation products or partial sequences, which comprises treating fibrin, a fibrin or fibrinogen degradation product or a fibrin or fibrinogen partial sequence with an oxidizing agent under conditions such that all or some of the methionine residues contained in the amino acid sequence of these substances are converted to methionine sulfoxide residues.

2. The process as claimed in claim 1, wherein the oxidizing agent is a halogenoamine, preferably chloramine.

3. The process as claimed in claim 1, wherein degradation products are used which have been prepared in the presence of chelate-forming agents.

4. The process as claimed in claim 1, wherein the degradation products or partial sequences contain the D domain or partial sequences thereof.

5. The process as claimed in claim 1, wherein a detergent is added to the reaction mixture.

6. The process as claimed in claim 1, wherein the oxidation is carried out at pH 7 - 9.5.

## Revendications (Revendications pour l'(les) Etat(s) contractant(s) suivant(s): AT, BE, CH, DE, FR, GB, IT, LI, LU, NL, SE)

1. Dérivé de la fibrine, produits de décomposition de la fibrine ou du fibrinogène ou séquences partielles de la fibrine ou du fibrinogène, caractérisés en ce que, dans les séquences d'aminoacides de base, les fragments méthionine sont transformés, en partie ou totalité, en fragments sulfoxyde de méthionine.

2. Dérivé selon la revendication 1, caractérisé en ce que les produits de décomposition ou séquences partielles contiennent le domaine D ou des séquences partielles de celui-ci.

3. Dérivé selon la revendication 1 en tant que médicament.

4. Fibrinogène dans lequel les fragments méthionine sont transformés, en partie ou en totalité, en sulfoxyde de méthionine, en tant que médicament.

5. Procédé pour préparer un dérivé selon la revendication 1, caractérisé en ce qu'on traite la fibrine, un produit de décomposition de la fibrine ou du fibrinogène ou une séquence partielle de la fibrine ou du fibrinogène, avec un agent d'oxydation, dans des conditions telles que les fragments méthionine que contient la séquence d'aminoacides de ces substances sont transformés, en partie ou en totalité, en fragments sulfoxyde de méthionine.

6. Procédé pour préparer un dérivé selon la revendication 5, où on utilise une halogéno-amine, de préférence une chloro-amine, comme agent d'oxydation.

7. Procédé selon la revendication 5, caractérisé en ce qu'on utilise des produits de décomposition qui ont été préparés en présence d'agents chélatants.

8. Procédé selon la revendication 5, caractérisé en ce qu'on ajoute un détergent au mélange réactionnel.

9. Procédé selon la revendication 5, caractérisé en ce qu'on effectue l'oxydation à un pH valant de 7 à 9,5.

10. Utilisation d'un dérivé selon la revendication 1 pour le diagnostic.

11. Utilisation d'un dérivé selon la revendication 1 comme agent affin .

12. Utilisation de fibrinogène, dans lequel les fragments méthionine ont été transformés, en partie ou en totalité, en fragments sulfoxyde de méthionine, comme agent affin .

## Revendications (Revendications pour l'(les) Etat(s) contractant(s) suivant(s): ES)

1. Procédé pour préparer un dérivé de la fibrine, des produits de décomposition de la fibrine ou du fibrinogène ou des séquences partielles de la fibrine ou du fibrinogène, caractérisé en ce qu'on traite la fibrine, un produit de décomposition de la fibrine ou du fibrinogène ou une séquence partielle de la fibrine ou du fibrinogène, avec un agent d'oxydation, dans des conditions telles que les fragments méthionine que contient la séquence d'aminoacides de ces substances sont transformés, en partie ou en totalité, en fragments sulfoxyde de méthionine.

2. Procédé selon la revendication 1, où on utilise une halogéno-amine, de préférence une chloro-amine, comme agent d'oxydation.

3. Procédé selon la revendication 1, caractérisé en ce qu'on utilise des produits de décomposition qui ont été préparés en présence d'agents chélatants.

4. Procédé selon la revendication 1, caractérisé en ce que les produits de décomposition ou séquences partielles contiennent le domaine D ou des séquences partielles de celui-ci.

5. Procédé selon la revendication 1, caractérisé en ce qu'on ajoute un détergent au mélange réactionnel.

6. Procédé selon la revendication 1, caractérisé en ce qu'on effectue l'oxydation à un pH valant de 7 à 9,5.
